# EUROPEAN PATENT APPLICATION

(11) **EP 3 431 111 A1**
(43) Date of publication of application: **23.01.2019**
(21) Application number: 18183850.9
(22) Date of filing: 17.07.2018
(51) Int. Cl.: A61L 27/54, A61L 31/16

(54) **DRUG ELUTING MEDICAL DEVICE**

(30) Priority: 18.07.2017 US 201762533932 P; 27.06.2018 US 201816019641
(71) Applicant: Covidien LP, Mansfield, MA 02048 (US)
(72) Inventor: HODGKINSON, Gerald, Guilford, CT Connecticut 06437 (US); SOLTZ, Michael, Fairfield, CT Connecticut 06825 (US); JACOBS, Emily, Hamden, CT Connecticut 06517 (US); SCHULZ-JANDER, Daniel, Oakland, CA California 94602 (US); HUSSAINI, Sulaiman, Santa Rosa, CA California CA (US)
(74) Representative: Maschio, Antonio

(57) **Abstract**

The present disclosure relates to medical devices, and methods for producing and using the devices. In embodiments, the medical device may be a buttress including an elongate rectangular body having a proximal portion, a distal portion, and opposing lateral sides running along the elongate rectangular body from the proximal portion to the distal portion. The substrate possesses a therapeutic layer of a therapeutic agent along at least one of its lateral sides. Methods and systems for producing such devices are also provided.

## Description

### CROSS-REFERENCE TO RELATED APPLICATIONS

This application claims the benefit of and priority to U.S. Provisional Patent Application No. 62/533,932 filed July 18, 2017, the entire disclosure of which is incorporated by reference herein.

### BACKGROUND

The present disclosure relates to medical devices, including surgical devices such as buttresses, for use with wound closure devices. Medical devices formed of the materials of the present disclosure are capable of delivering drugs to a patient.

Surgical stapling instruments are employed by surgeons to sequentially or simultaneously apply one or more rows of fasteners, e.g., staples or two-part fasteners, to body tissue for the purpose of joining segments of body tissue together. Such instruments generally include a pair of jaws or finger-like structures between which the body tissue to be joined is placed. When the stapling instrument is actuated, or "fired", longitudinally moving firing bars contact staple drive members in one of the jaws. The staple drive members push the surgical staples through the body tissue and into an anvil in the opposite jaw, which forms the staples. If tissue is to be removed or separated, a knife blade can be provided in the jaws of the device to cut the tissue between the lines of staples.

When stapling certain tissue, such as lung, esophageal, intestinal, duodenal, and vascular tissues, or relatively thin or fragile tissues, it may be desirable to seal the staple line against air or fluid leakage. Preventing or reducing air or fluid leakage can significantly decrease post-operative recovery time. Additionally, it may be desirable to reinforce the staple line against the tissue to prevent tears in the tissue or pulling of the staples through the tissue. One method of preventing these tears involves the placement of a biocompatible fabric reinforcing material, sometimes referred to herein, in embodiments, as a "buttress" material, between the staple and the underlying tissue.

For some surgical procedures, it may also be desirable to introduce therapeutic agents at the site of treatment.

Improved surgical repair materials, capable of use as buttresses for sealing and/or reinforcing staple lines against tissue, and improved methods for introducing therapeutic agents to a patient, remain desirable.

### SUMMARY

The present disclosure relates to medical devices, including surgical buttresses, which can be used with tissue fixation devices, and methods of using the same. Other medical devices not used with tissue fixation devices are contemplated as well, such as tissue supports or other structures.

In embodiments, a medical device of the present disclosure includes a substrate and a therapeutic layer on at least one edge of the porous substrate. In some embodiments, the medical device of the present disclosure includes a substrate including a generally rectangular body portion having a distal end and a proximal end, with opposing lateral sides that run along the rectangular body portion from the distal end to the proximal end, and a therapeutic layer on at least one of the lateral sides of the rectangular body portion of the substrate, such that the middle portion of the rectangular body portion of the substrate remains free of any therapeutic agent.

In embodiments, the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs , vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, and ribozymes.

In some embodiments, the therapeutic agent is a chemotherapy drug. Suitable chemotherapy drugs include, in embodiments, any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, and combinations thereof.

In some embodiments, the excipient includes a surfactant, a salt, an acid, a stabilizer, a polyhydric alcohol, a hydrotrope, a low molecular weight poly(ethylene glycol) or any combination thereof. Suitable surfactants include a cyclodextrin, sodium dodecyl sulfate, octyl glucoside, a sorbitan fatty acid ester, or combinations thereof. Suitable salts include sodium chloride. Suitable acids include oleic acid, citric acid, ascorbic acid, or combinations thereof. Suitable stabilizers include butylated hydroxytoluene. Suitable polyhydric alcohols include D-sorbitol, mannitol, or combinations thereof.

In embodiments, the medical device may be surgical buttresses, hernia patches, stents, and tissue scaffolds.

Methods for treating tissue are also provided, which include applying the medical device of the present disclosure to tissue.

Methods for producing the medical devices of the present disclosure are also provided. In embodiments, the method includes affixing a buttress to a cartridge of a stapler; placing the cartridge having the buttress thereon on a stage, the stage capable of movement in both x and y directions; supplying a needle with a solution including a therapeutic agent, a solvent, an optional carrier component, and an optional excipient; operating a processing unit having a user interface and a program for moving the stage in both x and y directions; and applying the solution to the buttress as the stage moves in both x and y directions so that the solution is applied to at least one edge of the buttress.

Systems for producing the medical devices are also provided. In embodiments, a system of the present disclosure includes a control unit attached to a stage, the stage capable of movement in both x and y directions and possessing a channel therein for placement of a staple cartridge therein; a needle attached to a source of a solution; and a processing unit having a user interface and a program for determination of set values for movement of the stage in both x and y directions and dispensing the solution onto a buttress affixed to the staple cartridge.

### BRIEF DESCRIPTION OF THE DRAWINGS

Embodiments of the presently disclosed specimen retrieval device are described herein with reference to the drawings wherein:
FIG. 1 is a top view of a buttress that has been treated in accordance with an embodiment of the present disclosure, showing the application of a therapeutic layer along the edges of the buttress, including at least one therapeutic agent therein;
FIG. 2 is an alternate top view of a buttress having a different geometry that may be treated in accordance with an embodiment of the present disclosure;
FIG. 3 is an alternate top view of a buttress having a different geometry that may be treated in accordance with an embodiment of the present disclosure;
FIG. 4 is a depiction of a buttress of the present disclosure attached to a cartridge of a stapler, prior to application of a therapeutic coating at an edge of the buttress in accordance with a method of the present disclosure;
FIG. 5 is a depiction of a system for application of a therapeutic layer to the buttress depicted in FIG. 4, showing both a needle for application of a therapeutic coating at an edge of the buttress, and a stage permitting movement of the cartridge and buttress to apply the coating in desired areas of the buttress, in accordance with a method of the present disclosure; and
FIG. 6 is a top view of the buttress of FIG. 3, showing the application of a therapeutic layer along the edges of the buttress, including at least one therapeutic agent therein.

### DETAILED DESCRIPTION

Various exemplary embodiments of the present disclosure are discussed herein below in terms of buttresses for use with tissue fixation devices, in embodiments surgical staples. While the below disclosure discusses in detail the use of these buttresses with staples, it will be appreciated that medical devices of the present disclosure include a range of buttressing materials and film-based medical devices that are used to mechanically support tissues, reinforce tissues along staple or suture lines, and decrease the incidence of fluid leakage and/or bleeding of tissues. For example, other suitable medical devices include hernia patches, stents, and/or tissue scaffolds.

Medical devices of the present disclosure may be used with any fixation device utilized to close any wound, defect, and/or opening in tissue. Thus, while surgical buttresses are discussed in conjunction with a surgical stapling apparatus, it is envisioned that other fixation devices, such as tacks, sutures, clips, adhesives, and the like, may be utilized in conjunction with medical devices of the present disclosure to affix the medical devices to tissue. Medical devices that are not used with a tissue fixation device, or other tissue support devices, are also contemplated.

The buttress of the present disclosure is in the form of a generally rectangular body having a distal end and a proximal end, with opposing lateral sides that run along the length of the elongate rectangular body portion from the distal end to the proximal end. In embodiments the medical devices of the present disclosure include therapeutic agent(s) in a therapeutic layer formed along at least one lateral side, or edge, of the buttress. In other embodiments, the buttress has two therapeutic layers, one along each lateral side, or edge, of the buttress.

Therapeutic agents included in therapeutic layers along the edges of the buttress of the present disclosure are suitable for further treatment of tissue at or near the site where the surgical buttress of the present disclosure is placed. Thus, the present disclosure describes surgical buttresses, and methods and mechanisms for using the same, for the targeted delivery of therapeutic agents to a patient.

In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse or other care provider and may include support personnel.

Medical devices of the present disclosure, including surgical buttresses, may be fabricated from a biocompatible substrate material. Such substrates may be formed of bioabsorbable, non-absorbable, natural and/or synthetic materials.

In embodiments, the medical devices of the present disclosure, such as a surgical buttress, may be biodegradable, so that the device does not have to be retrieved from the body. The term "biodegradable" as used herein is defined to include both bioabsorbable and bioresorbable materials. By biodegradable, it is meant that the medical device decomposes or loses structural integrity under body conditions (e.g., enzymatic degradation or hydrolysis), or is broken down (physically or chemically) under physiologic conditions in the body such that the degradation products are excretable or absorbable by the body.

Non-limiting examples of materials which may be used in forming a medical device of the present disclosure, for example a surgical buttress, include, but are not limited to, poly(lactic acid), poly(glycolic acid), poly(trimethylene carbonate), poly(dioxanone), poly(hydroxybutyrate), poly(phosphazine), polyethylene terephthalate, polyethylene glycols, polyethylene oxides, polyacrylamides, polyhydroxyethylmethylacrylate, polyvinylpyrrolidone, polyvinyl alcohols, polyacrylic acid, polyacetate, polycaprolactone, polypropylene, aliphatic polyesters, glycerols, poly(amino acids), copoly(ether-esters), polyalkylene oxalates, polyamides, poly(iminocarbonates), polyalkylene oxalates, polyoxaesters, polyorthoesters, polyphosphazenes, and copolymers, block copolymers, homopolymers, blends and combinations thereof.

In embodiments, natural biological polymers may be used in forming a medical device of the present disclosure. Suitable natural biological polymers include, but are not limited to, collagen, gelatin, fibrin, fibrinogen, elastin, keratin, albumin, cellulose, oxidized cellulose, hydroxyethyl cellulose, hydroxypropyl cellulose, carboxyethyl cellulose, carboxymethyl cellulose, chitin, chitosan, and combinations thereof. In addition, natural biological polymers may be combined with any of the other polymeric materials described herein to produce a medical device of the present disclosure.

The medical device may also be formed of materials that are porous or non-porous. It should of course be understood that any combination of porous, non-porous, natural, synthetic, bioabsorbable, and/or non-bioabsorbable materials may be used to form a medical device of the present disclosure.

In some embodiments, a medical device of the present disclosure, such as a surgical buttress, may be formed of porous material(s). Any porous portion of a medical device of the present disclosure may have openings or pores over at least a part of a surface thereof. Suitable porous materials include, but are not limited to, fibrous structures (e.g., knitted structures, woven structures, non-woven structures, etc.) and/or foams (e.g., open or closed cell foams).

In embodiments, the pores may be in sufficient number and size so as to interconnect across the entire thickness of the medical device. Woven fabrics, knitted fabrics and open cell foams are illustrative examples of structures in which the pores can be in sufficient number and size so as to interconnect across the entire thickness of the medical device.

In other embodiments, the pores may not interconnect across the entire thickness of the medical device. Closed cell foams or fused non-woven materials are illustrative examples of structures in which the pores may not interconnect across the entire thickness of the medical device. In some embodiments, pores may be located on a portion of the medical device, with other portions of the medical device having a non-porous texture. Those skilled in the art may envision a variety of pore distribution patterns and configurations for a porous medical device of the present disclosure.

Where the medical device of the present disclosure is porous and includes fibrous materials, the medical device may be formed using any suitable method including, but not limited to, knitting, weaving, non-woven techniques (including melt blowing), wet-spinning, electro-spinning, extrusion, co-extrusion, and the like. In embodiments, the medical device is a surgical buttress possessing a three dimensional structure, such as the textiles described in U.S. Patent Nos. 7,021,086 and 6,443,964, the entire disclosures of each of which are incorporated by reference herein.

The porosity of the fabric used to form the substrate may allow for the infiltration of biological fluids and/or cellular components which, in turn, may accelerate the release kinetics of any therapeutic agent from the medical device of the present disclosure, thus increasing the rate of release of therapeutic agent(s) from the medical device into the surrounding tissue and fluids.

Substrates used to form medical devices of the present disclosure, such as surgical buttresses, may have a thickness from about 0.05 mm to about 0.5 mm, in embodiments from about 0.1 mm to about 0.2 mm.

Where the substrate used to form the medical device is porous, the medical device of the present disclosure may have a pore volume from about 65% to about 85%, in embodiments from about 70% to about 80%.

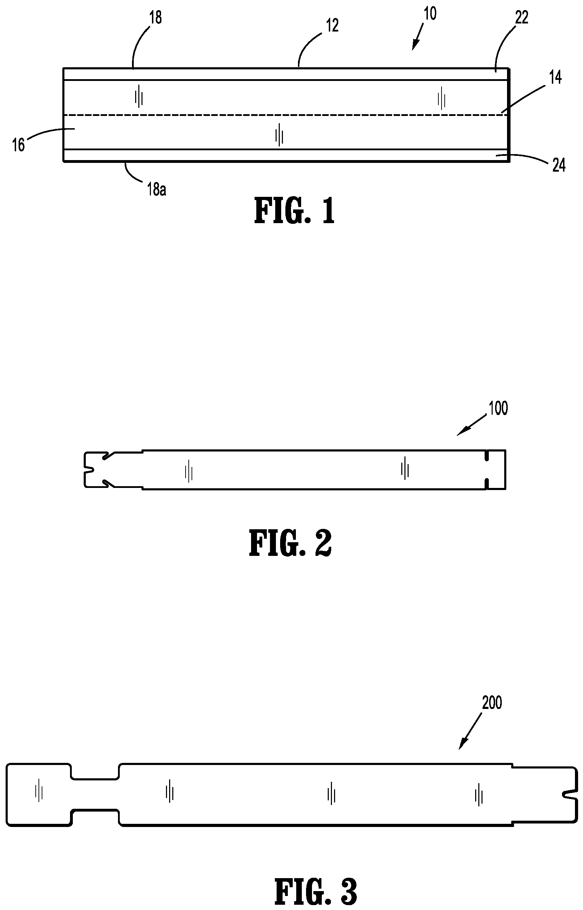

Turning to the Figures, as depicted in FIG. 1, in embodiments, a buttress 10 of the present disclosure may include an elongate rectangular body portion 12. The rectangular body of the buttress has a distal portion 14 and a proximal portion 16, with opposing lateral sides 18, 18a, that run along the length of the elongate rectangular body portion 12 from the distal portion 14 to the proximal portion 16. These lateral portions 18, 18a may, in embodiments, be referred to as "edges." In some embodiments, the buttress 10 of the present disclosure has a therapeutic layer along at least one edge. In other embodiments, as depicted in FIG. 1, the buttress has two therapeutic layers 22 and 24, one along each edge 18, 18a of the rectangular body portion 12 of the buttress 10 of the present disclosure.

While the above description is directed to rectangular buttresses, it is to be appreciated that any suitable configuration for a buttress may be utilized in accordance with the present disclosure. For example, buttresses having an elongate rectangular body with head and tail portions at the ends of the buttress may be utilized. Examples of such buttresses are included in FIGs. 2 and 3, with FIG. 2 depicting alternate buttress 100 and FIG. 3 depicting alternate buttress 200. Any other suitable shape may be utilized. For example, additional suitable buttresses include those disclosed in U.S. Patent Application Serial No. 15/639,367, filed June 30, 2017, and U.S. Patent Nos. 8,157,151, 8,561,873 and 9,693,772, the entire disclosures of each of which are incorporated by reference herein.

Therapeutic agents which may be added to a medical device of the present disclosure include, but are not limited to, drugs, amino acids, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor), vaccines (e.g., tumoral, bacterial and viral antigens), somatostatin, antigens, blood coagulation factors, growth factors (e.g., nerve growth factor, insulin-like growth factor), bone morphogenic proteins, TGF-B, protein inhibitors, protein antagonists, protein agonists, nucleic acids, such as antisense molecules, DNA, RNA, RNAi, oligonucleotides, polynucleotides, cells, viruses, and ribozymes.

In embodiments, the therapeutic agent applied to a medical device of the present disclosure may include an anti-tumor agent and/or tumor suppressor, referred to, in embodiments, as a "chemotherapeutic agent" and/or an "antineoplastic agent." Suitable chemotherapeutic agents include, for example, paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine (BCNU), methyl-CCNU, cisplatin, etoposide, camptothecin and derivatives thereof, phenesterine, vinblastine, vincristine, goserelin, leuprolide, interferon alfa, retinoic acid (ATRA), nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, combinations thereof, and the like.

In embodiments, paclitaxel and/or paclitaxel derivatives may be used as the therapeutic agent. Paclitaxel may have various forms, referred to herein as "polymorphs," including amorphous paclitaxel, crystalline paclitaxel, sometimes referred to as crystalline paclitaxel dihydrate, and/or anhydrous paclitaxel, or mixtures thereof.

In accordance with the present disclosure, the polymorph form of paclitaxel utilized in forming the therapeutic layer may be varied by the aqueous composition, the solvent polarity and the composition of protic and aprotic solvents utilized in the solvent system to form the solution for applying the therapeutic layer. For example, paclitaxel dissolved and then dried from 10% v/v water in methanol will yield a predominantly crystalline paclitaxel dihydrate layer, while the same paclitaxel dissolved and then dried from non-polar solvent dichloromethane will yield a predominantly amorphous layer.

The crystallinity of the paclitaxel will impact its solubility in aqueous systems. Accordingly, the polymorph form of paclitaxel in the therapeutic layer may be adjusted and selected to provide a tailored release of therapeutic agent from the implant of the present disclosure. Although the drug in any form is hydrophobic, as amorphous paclitaxel it is more soluble in aqueous environments, and crystalline paclitaxel is less soluble in aqueous environments, more than one polymorphic form of paclitaxel may be used, in embodiments, to provide implants that have multiple release profiles of paclitaxel. For example, medical devices of the present disclosure having both amorphous paclitaxel and crystalline paclitaxel dihydrate thereon may release a bolus of therapeutic agent upon implantation (in the form of the amorphous paclitaxel), while also slowly releasing the therapeutic agent (in the form of the crystalline paclitaxel dihydrate).

In embodiments with no excipient, the amount of amorphous paclitaxel in the therapeutic layer on the medical device may be from 0 % to about 100 % by weight of the therapeutic layer, in embodiments from about 10 % to about 90 % by weight of the therapeutic layer, with the crystalline paclitaxel dihydrate being present in amounts from about 0 to about 100% by weight of the therapeutic layer, in embodiments from about 90 % to about 10 % by weight of the therapeutic layer.

Medical devices of the present disclosure may release amorphous paclitaxel over a period of time from about 24 hours to about 168 hours, in embodiments from about 48 hours to about 96 hours, and release the crystalline paclitaxel dihydrate over a period of time from about 1 week to about 6 weeks, in embodiments from about 2 weeks to about 4 weeks.

In some embodiments, the therapeutic layer along at least one edge of the buttress may be formed of polymeric materials or other carrier components within the purview of those skilled in the art. In embodiments, such layers may include, for example, degradable materials such as those prepared from monomers such as glycolide, lactide, trimethylene carbonate, p-dioxanone, epsilon-caprolactone, and combinations thereof.

In other embodiments, regardless of whether the therapeutic agent is applied with or without some additional polymeric material to form the therapeutic layer, in addition to the therapeutic agents described above, therapeutic layers applied to the substrate material in forming a medical device of the present disclosure may also include excipients to enhance both the ability of the therapeutic agent to adhere to the medical device, in embodiments a surgical buttress, as well as to modify the elution of the therapeutic agent from the medical device.

In embodiments, suitable excipients which may be combined with a therapeutic agent to form the therapeutic layer on the medical device include surfactants such as, but not limited to, cyclodextrins such as 2-hydroxypropyl-beta-cyclodextrin and methyl-β-cyclodextrin, sodium dodecyl sulfate, octyl glucoside, and sorbitan fatty acid esters such as sorbitan monooleate, sorbitan monolaurate and polyethoxylated fatty acid esters of sorbitan, sometimes referred to herein as polysorbates, including those sold under the name TWEEN™. Examples of such polysorbates include polysorbate 80 (TWEEN™ 80), polysorbate 20 (TWEEN™ 20), polysorbate 60 (TWEEN™ 60), polysorbate 65 (TWEEN™ 65), polysorbate 85 (TWEEN™ 85), combinations thereof, and the like. In embodiments, low molecular weight poly(ethylene glycol)s may be added as an excipient, either alone or in any combination with any of the other above excipients.

In other embodiments, suitable excipients may include salts such as sodium chloride and/or other materials such as urea, oleic acid, citric acid, and ascorbic acid. In yet other embodiments, the excipient may be a stabilizer such as butylated hydroxytoluene (BHT).

Still other suitable excipients include polyhydric alcohols such as D-sorbitol, mannitol, combinations thereof, and the like.

In some embodiments, excipients which are hydrotropes may be included in the therapeutic layers of the present disclosure. These materials attract water into the therapeutic layer, which may enhance its degradation and resulting release of the therapeutic agent from the therapeutic layer.

In embodiments, the therapeutic agent(s), carrier component(s) and/or excipient(s) may be in a solution for application to a medical device of the present disclosure. Any suitable biocompatible solvent may be used to form such a solution. Suitable solvents for forming such a solution include any pharmaceutically acceptable solvents including, but not limited to, saline, water, alcohol, acetone, dimethyl sulfoxide, ethyl acetate, N-methylpyrrolidone, combinations thereof, and the like. Methods for forming such solutions are within the purview of those skilled in the art and include, but are not limited to, mixing, blending, sonication, heating, combinations thereof, and the like.

In embodiments, by selecting different solvent systems, different dissolution rates of the therapeutic agent(s) may be achieved due to different therapeutic agent morphologies and degrees of crystallinity that occur based upon the solvent used in forming the solution including the therapeutic agent(s).

In embodiments, the therapeutic agent(s), any carrier component(s), and/or any excipient(s) may be applied to the edges of a medical device of the present disclosure by a needle deposition process. As noted above, in embodiments the therapeutic agent is in a solution, which is then applied to a medical device of the present disclosure, such as a buttress. The solution possessing the therapeutic agent, along with any carrier component(s), and/or any excipient(s), may be applied to the edges of a medical device as follows.

In embodiments, the therapeutic agent(s), any carrier component(s), and/or any excipient(s) may be applied to the edges of a medical device of the present disclosure prior to affixing the medical device to some other medical apparatus. For example, in the case of a buttress, the buttress may be coated in accordance with the present disclosure prior to its attachment to a surgical stapler.

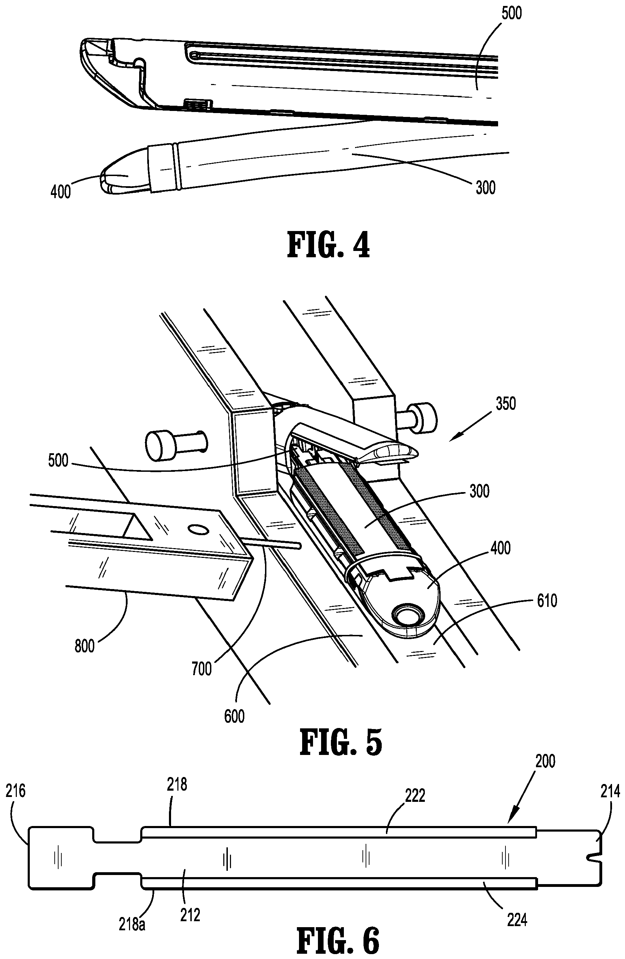

In other embodiments, as depicted in FIGs. 4 and 5, the medical device of the present disclosure may be affixed to another medical apparatus, and then coated in accordance with the present disclosure. For example, in the case of a buttress, the buttress may be coated in accordance with the present disclosure after its attachment to a surgical stapler. FIG. 4 shows a buttress 300 attached to a cartridge 400 of a stapler 500.

FIG. 5 depicts an embodiment of the present disclosure encompassing a system 350 for forming the medical devices of the present disclosure. As depicted in FIG. 5, a buttress 300 of the present disclosure is affixed to a cartridge 400 of a stapler 500 and coated in accordance with the present disclosure. Briefly, cartridge 400 is placed on a stage 600 within a channel 610 in the stage 600, the stage 600 being capable of movement in both x and y directions. A stainless steel needle 700 is attached to an arm 800 and connected to a source of solution (not shown) including the therapeutic agent(s), solvent(s), any carrier component(s), and/or any excipient(s). The needle 700 is hollow, and capable of introducing the solution onto a defined surface of the buttress 300. In embodiments (not shown), the needle 700 interfaces at a specified angle with the buttress 300 and dispenses the solution possessing the therapeutic agent(s), solvent(s), any carrier component(s), and/or any excipient(s) via a custom written software program. The system includes a processing unit (not shown) that runs software program and has a user interface for the input of set values for movement of the stage in both x and y directions, as well as the dispensing of the solution possessing the therapeutic agent(s), solvent(s), any carrier component(s), and/or any excipient(s) onto the buttress 300. The system 350 includes a control unit (not shown) attached to the stage 600, which moves the stage 600 in both x and y directions. Another control unit (not shown) controls the dispensing of the solution onto the buttress 300.

In other embodiments, instead of a needle, inkjet printing techniques, deposition methods including piezoelectric elements, combinations thereof, and the like, may be used to apply the therapeutic layer(s).

FIG. 6 depicts the buttress of FIG. 3 coated in accordance with the present disclosure. The buttress 200, has elongate body portion 212. The elongate body portion 212 of the buttress 200 has a distal portion 214 and a proximal portion 216, with opposing lateral sides 218, 218a, that run along the length of the elongate body portion 212 from the distal portion 214 to the proximal portion 216. The buttress 200 has two therapeutic layers 222 and 224, one along each edge 218, 218a of the buttress 200 of the present disclosure.

While the therapeutic layers 222 and 224, one along each edge 218, 218a of the buttress 200 of the present disclosure, have been depicted as stripes or continuous edges, it is to be appreciated that the therapeutic layers may possess varying geometries (not shown), including interrupted stripes, rectangles, semi-circles, triangles, squares, combinations thereof, and the like.

In accordance with the present disclosure, the therapeutic layer, including the therapeutic agent(s), solvent(s), any carrier component(s), and/or any excipient(s), is applied so that an adequate amount of therapeutic agent(s) is deposited and stays robustly attached along the edges of the buttress, while a wide middle portion of the buttress remains free of any therapeutic agent(s).

After application, the solvent from the coating solution may be driven off by methods within the purview of those skilled in the art. For example, solvent evaporation may be facilitated by heat, gas flow, time, reduced pressure, combinations thereof, and the like, to increase the accuracy of drug deposition on the medical device. Moreover, this assisted evaporation of solvent may be applied to the whole surface of the medical device, partially to only a portion of the surface of the medical device, or just around the deposition instrument (e.g., a needle tip).

Driving off the solvent leaves the therapeutic agent and any carrier component and/or excipient behind to form the therapeutic layer at the edges of the medical device.

In accordance with the present disclosure, the process may be repeated, such that multiple passes can be made so that the edges of the medical device have the desired amount of therapeutic agent for administering a dose of the therapeutic agent. In embodiments, repeating the process described above results in the deposition of multiple layers such that the overall therapeutic layer at the edges of the buttress is very uniform and robust, and adheres to the buttress material very well. This is in contrast to other processes, such as dip coating and other similar coating methods, which lack both the robustness and adherence of the coatings/layers produced in accordance with the present disclosure.

The process is designed in such a way that it leverages the capillary action of the fabric on which the drug is dispensed for coating. The speed and rate of dispersion are appropriately controlled to produce the desired coatings. Between coatings, the process may have pre-determined pauses to ensure each coat has the proper time to dry before more therapeutic agent is deposited as a next layer.

Utilizing the processes of the present disclosure, there is limited drug loss during the different stages of the process. This is beneficial in terms of isolating the therapeutic agent to areas where it is intended to stay, and cost savings in terms of the amount of therapeutic agent being used.

The process is very efficient from a manufacturing aspect as well. Using the process of the present disclosure, one can coat directly on the buttress rather than coat on cartridge and anvil assemblies, which saves with respect to material handling, labor costs and quality related costs. Moreover, it is much safer for the operators. For example, the process is developed such that it is better than spray coating which can be very hazardous for operators running the process. A normal air flow hood would suffice for operating this process safely. Also, an isolator is not needed for this process which makes it very ergonomic for long term manufacturing.

The process is also designed such that it has the capability to deposit drug on specific areas on the device with high precision. The resolution of the process, i.e., the distance at which the therapeutic layer may be applied from the edge of the medical device, is from about 0.1 mm to about 10 mm, in embodiments from about 0.5 mm to about 2 mm. Certain sections of the surface of the device can be left non-coated by design to improve the performance of the medical device, for instance better tissue healing around the staple line.

In embodiments, multiple layers of therapeutic agents can be deposited on the medical device with ease. In some cases, different therapeutic agents are applied in different layers. Different therapeutic benefits can thus be combined on one device by using the multiple layers. In other embodiments, different therapeutic agents can be deposited on different areas on the surface of the medical device, e.g., one therapeutic agent can be applied in one region/area, and a different therapeutic agent can be applied to a different region/area.

After formation, medical devices of the present disclosure may possess the therapeutic agent in the coated buttress thereon in amounts from about 0.1 % to about 50 % by weight of the coated buttress, in embodiments from about 1 % to about 10 % by weight of the coated buttress. While excipients are not required, where present, non-polymeric excipients may be present in an amount from about 0.01 % to about 80% by weight of the coated buttress, in embodiments from about 1 % to about 11 % by weight of the coated buttress. In other embodiments, where present, polymeric excipients may be present in an amount from about 0.014% to about 14% by weight of the coated buttress, in embodiments from about 5% to about 15% by weight of the coated buttress.

After formation, medical devices of the present disclosure may possess the therapeutic agent in the therapeutic layer thereon in amounts from about 0.01 % to about 100 % by weight of the therapeutic layer, in embodiments from about 1% to about 75 % by weight of the therapeutic layer. While excipients are not required, where present, non-polymeric excipients may be present in an amount from about 1% to about 99% by weight of the therapeutic layer, in embodiments from about 8.5% to about 79.4% by weight of the therapeutic layer, and most preferably in embodiments from 9.5% to about 15%. In embodiments, where present, polymeric excipients may be present in an amount from about 1% to about 99% by weight of the therapeutic layer, in embodiments from about 5% to about 15% by weight of the therapeutic layer.

A therapeutic layer having both a therapeutic agent and non-polymeric excipients may have a thickness from about 13 nm to about 2.9 µm, in embodiments from about 25 nm to about 100 nm.

A therapeutic layer having both a therapeutic agent and polymeric excipients may have a thickness from about 2 nm to about 1.1 µm, in embodiments from about 30 nm to about 100 nm.

In other embodiments, the therapeutic layers may include little or no excipients, so very thin therapeutic layers may be applied to the substrate. This will maintain the porosity of the substrate. Such therapeutic layers may have a thickness from about 11 nm to about 218 nm, in embodiments from about 25 nm to about 75 nm.

Medical devices of the present disclosure may release therapeutic agents therefrom over a period of time from about 24 hours to about 4 weeks, in embodiments from about 48 hours to about 2 weeks.

As noted above, the medical device of the present disclosure may be used with any fixation device to further assist in sealing tissue. For example, medical devices of the present disclosure may be used in conjunction with staples, tacks, clips, sutures, adhesives, combinations thereof, and the like.

In embodiments, medical devices of the present disclosure may be used with staples. For example, a surgical buttress formed of a medical device of the present disclosure is provided to reinforce and seal the lines of staples applied to tissue by a surgical stapling apparatus. The buttress may be configured into any shape, size, or dimension suitable to fit any surgical stapling, fastening, or firing apparatus.

In embodiments, the buttresses described herein may be used in sealing a wound by approximating the edges of wound tissue between a staple cartridge and an anvil of a surgical stapling apparatus which contains the buttress. Firing of the surgical stapling apparatus forces the legs of at least one staple to pass through the opening on the staple cartridge and the buttress, the tissue, and the openings on the anvil to secure the buttress to the tissue, to secure the adjoining tissue to one another, and to seal the tissue.

Where the medical device of the present disclosure is used to form a surgical buttress, upon application to a site of bleeding tissue, the buttress may affect hemostasis of said tissue. As used herein, the term "hemostasis" means the arrest of bleeding.

In addition to providing hemostasis at the site of application of the buttress, the medical devices of the present disclosure may also provide for treatment of tissue with the therapeutic agent at both the site of implantation and elsewhere in the body.

It will be understood that various modifications may be made to the embodiments disclosed herein. Therefore, the above description should not be construed as limiting, but merely as an exemplification of preferred embodiments. Those skilled in the art will envision other modifications within the scope and spirit of the present disclosure. Such modifications and variations are intended to come within the scope of the following claims.

The invention may be described by reference to the following numbered paragraphs:-
1. A medical device comprising:
   a substrate including a generally rectangular body portion having a distal end and a proximal end, with opposing lateral sides that run along the rectangular body portion from the distal end to the proximal end; and
   a therapeutic layer on at least one of the lateral sides of the rectangular body portion of the substrate, such that the middle portion of the rectangular body portion of the substrate remains free of any therapeutic agent.
2. The medical device of paragraph 1, wherein the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs , vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, and ribozymes.
3. The medical device of paragraph 1, wherein the therapeutic agent is a chemotherapy drug.
4. The medical device of paragraph 3, wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, and combinations thereof.
5. The medical device of paragraph 1, wherein the excipient includes a surfactant, a salt, an acid, a stabilizer, a polyhydric alcohol, a hydrotrope, a low molecular weight poly(ethylene glycol) or any combination thereof.
6. The medical device of paragraph 5, wherein the surfactant is a cyclodextrin, sodium dodecyl sulfate, octyl glucoside, a sorbitan fatty acid ester, or combinations thereof.
7. The medical device of paragraph 5, wherein the salt includes sodium chloride.
8. The medical device of paragraph 5, wherein the acid includes oleic acid, citric acid, ascorbic acid, or combinations thereof.
9. The medical device of paragraph 5, wherein the stabilizer includes butylated hydroxytoluene.
10. The medical device of paragraph 5, wherein the polyhydric alcohol includes D-sorbitol, mannitol, or combinations thereof.
11. The medical device of paragraph 1, wherein the medical device is selected from the group consisting of surgical buttresses, hernia patches, stents, and tissue scaffolds.
12. A method for treating tissue comprising applying the medical device of paragraph 1 to tissue.
13. A method comprising:
   affixing a buttress to a cartridge of a stapler;
   placing the cartridge having the buttress thereon on a stage, the stage capable of movement in both x and y directions;
   supplying a needle with a solution including a therapeutic agent, a solvent, an optional carrier component, and an optional excipient;
   operating a processing unit having a user interface and a program for moving the stage in both x and y directions; and
   applying the solution to the buttress as the stage moves in both x and y directions so that the solution is applied to at least one edge of the buttress.
14. The method of paragraph 13, wherein the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs , vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, and ribozymes.
15. The method of paragraph 13, wherein the therapeutic agent is a chemotherapy drug.
16. The method of paragraph 15, wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, and combinations thereof.
17. The method of paragraph 13, wherein the excipient includes a surfactant, a salt, an acid, a stabilizer, a polyhydric alcohol, a hydrotrope, a low molecular weight poly(ethylene glycol) or any combination thereof.
18. A system comprising:
   a control unit attached to a stage, the stage capable of movement in both x and y directions and possessing a channel therein for placement of a staple cartridge therein;
   a needle attached to a source of a solution;
   and a processing unit having a user interface and a program for determination of set values for movement of the stage in both x and y directions and dispensing the solution onto a buttress affixed to the staple cartridge.

## Claims

1. A medical device comprising:
a substrate including a generally rectangular body portion having a distal end and a proximal end, with opposing lateral sides that run along the rectangular body portion from the distal end to the proximal end; and
a therapeutic layer on at least one of the lateral sides of the rectangular body portion of the substrate, such that the middle portion of the rectangular body portion of the substrate remains free of any therapeutic agent.

2. The medical device of claim 1, wherein the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs , vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, and ribozymes.

3. The medical device of claim 1 or claim 2, wherein the therapeutic agent is a chemotherapy drug.

4. The medical device of claim 3, wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, and combinations thereof.

5. The medical device of any preceding claim, wherein the excipient includes a surfactant, a salt, an acid, a stabilizer, a polyhydric alcohol, a hydrotrope, a low molecular weight poly(ethylene glycol) or any combination thereof.

6. The medical device of claim 5, wherein the surfactant is a cyclodextrin, sodium dodecyl sulfate, octyl glucoside, a sorbitan fatty acid ester, or combinations thereof.

7. The medical device of claim 5 or claim 6, wherein the salt includes sodium chloride.

8. The medical device of any of claims 5 to 7, wherein the acid includes oleic acid, citric acid, ascorbic acid, or combinations thereof.

9. The medical device of any of claims 5 to 8, wherein the stabilizer includes butylated hydroxytoluene.

10. The medical device of any of claims 5 to 9, wherein the polyhydric alcohol includes D-sorbitol, mannitol, or combinations thereof.

11. The medical device of any preceding claim, wherein the medical device is selected from the group consisting of surgical buttresses, hernia patches, stents, and tissue scaffolds.

12. A method comprising:
affixing a buttress to a cartridge of a stapler;
placing the cartridge having the buttress thereon on a stage, the stage capable of movement in both x and y directions;
supplying a needle with a solution including a therapeutic agent, a solvent, an optional carrier component, and an optional excipient;
operating a processing unit having a user interface and a program for moving the stage in both x and y directions; and
applying the solution to the buttress as the stage moves in both x and y directions so that the solution is applied to at least one edge of the buttress.

13. The method of claim 12, wherein the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs , vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, and ribozymes.

14. The method of claim 12 or claim 13, wherein the therapeutic agent is a chemotherapy drug; preferably wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, and combinations thereof; and/or wherein the excipient includes a surfactant, a salt, an acid, a stabilizer, a polyhydric alcohol, a hydrotrope, a low molecular weight poly(ethylene glycol) or any combination thereof.

15. A system comprising:
a control unit attached to a stage, the stage capable of movement in both x and y directions and possessing a channel therein for placement of a staple cartridge therein;
a needle attached to a source of a solution;
and a processing unit having a user interface and a program for determination of set values for movement of the stage in both x and y directions and dispensing the solution onto a buttress affixed to the staple cartridge.
